# DEMANDE DE BREVET EUROPEEN

(11) **EP 1 157 715 A1**
(43) Date de publication de la demande: **28.11.2001**
(21) Numéro de dépôt: 01401230.6
(22) Date de dépôt: 14.05.2001
(51) Int. Cl.: A61M 16/06, A61B 5/113

(54) **Masque respiratoire nasal avec thermistance réglable**

(30) Priorité: 26.05.2000 FR 0006773
(71) Demandeur: TAEMA, F-92182 Antony Cédex (FR)
(72) Inventeur: Billette de Villemeur, Pierre, 78150 Le Chesnay (FR); Thouvier, Stéphane, 95100 Argenteuil (FR)
(74) Mandataire: Pittis, Olivier

(57) **Abrégé**

L'invention concerne une interface (1,11) respiratoire faciale comprenant un corps (2,12) d'interface portant au moins une thermistance (3) ayant une extrémité active (4) susceptible de venir se placer en regard de la bouche d'un utilisateur lorsque l'interface (1,11) est mise en position sur la région faciale dudit utilisateur, caractérisée en ce que la longueur (L) séparant l'extrémité active (4) du corps (2,12) d'interface est ajustable.

L'interface selon l'invention est utilisable dans le cadre d'un traitement de troubles respiratoires chez un patient, en particulier de l'apnée du sommeil.

## Description

La présente invention concerne une interface respiratoire faciale, tel un masque respiratoire nasal ou des lunettes respiratoires, susceptible d'être utilisée pour diagnostiquer, soigner ou prévenir les troubles respiratoires chez un utilisateur, telle l'apnée du sommeil.

Actuellement, les masques respiratoires ou autres interfaces faciales respiratoires sont communément utilisés à des fins diverses et variées, notamment pour administrer de l'oxygène ou des mélanges d'air enrichi en oxygène aux personnes souffrant de problèmes pulmonaires; pour administrer des gaz anesthésiques pendant la phase préopératoire ; pour administrer de l'air sous pression aux personnes souffrant de troubles respiratoires, par exemple l'apnée du sommeil ; dans le cadre d'un traitement du type CPAP (Continous Positive Airway Pressure = Pression Positive Continue des Voies Respiratoires) ou d'un traitement mettant en oeuvre deux niveaux de pression.

En d'autres termes, les masques et les lunettes respiratoires permettent de faire l'interface entre les appareils médicaux de ventilation barométriques et/ou volumétriques et les patients devant être alimentés en gaz respiratoire.

Habituellement, les masques respiratoires sont fabriqués par moulage de matériaux plastiques et conformés pour s'adapter aux contours de la région nasale de l'utilisateur.

En fait, il existe deux types principaux de masques respiratoires nasaux, à savoir ceux recouvrant la région nasale et ceux comportant des embouts qui s'appliquent au contact des narines à la manière de canules.

En fait, ces deux types de masques diffèrent principalement par la manière dont est réalisée l'étanchéité. Ainsi, dans le premier cas l'étanchéité est réalisée autour du nez, alors que dans le second cas l'étanchéité est réalisée au niveau de chaque narine.

Les masques à embouts nasaux présentent l'avantage d'éviter tout contact avec l'arête du nez du patient, laquelle arête est une zone habituellement très sensible à l'appui des masques industriels. Cependant, les masques de ce type sont difficiles à maintenir en bonne position du fait de la faible surface de contact avec le visage, laquelle est limitée à une partie de la région interne des narines.

En outre, les masques recouvrant le nez sont les plus utilisés. Ceux-ci sont, en général, réalisés en un matériau polymère, tel un chlorure de vinyle ou du silicone, étant donné que ces matières permettent d'éviter ou de minimiser les effets irritant du masque pour la peau du patient.

Selon le masque, la disposition de l'arrivée de gaz peut varier mais la majorité d'entre eux comporte un orifice central d'alimentation en gaz dont le raccordement au circuit d'alimentation en gaz de l'appareil d'assistance respiratoire ou de la source de gaz sous pression est réalisé à l'aide d'un connecteur ou d'un dispositif analogue. A ce titre, on peut citer les documents US-A-4,655,213, US-A-3,580,051 et US-A-5,117,819.

Parfois, le masque peut comprendre plusieurs orifices d'alimentation en gaz, ainsi que décrit dans le document US-A-4,944,310.

En outre, pour obtenir une meilleure étanchéité du masque, lorsque celui-ci est en position sur le visage de l'utilisateur, le document EP-A-0462701 propose un masque nasal comprenant une coquille conformée pour recouvrir la région nasale de l'utilisateur, sur laquelle coquille est montée une membrane en un matériau élastomère capable de se distendre pour définir une chambre ayant une région terminale extérieurement convexe à parois mince pouvant se gonfler vers l'extérieur lorsque le masque est en service; c'est-à-dire lorsqu'il est placé en position sur le nez de l'utilisateur.

Une alternative à ce type de masque est proposé par les documents WO-A-97/09090 qui décrit un masque comportant une coquille destinée à recevoir la région nasale de l'utilisateur, l'étanchéité entre ladite coquille et le visage de l'utilisateur étant obtenue grâce à la présence, sur le contour de la coquille en contact avec le visage du patient, d'une chambre périphérique remplie d'un gel.

Par ailleurs, les lunettes respiratoires se composent, quant à elles, habituellement d'une portion tubulaire creuse acheminant du gaz respiratoire et munie de deux embouts ou canules destinés à venir se positionner dans les narines de l'utilisateur de manière à pouvoir y administrer du gaz d'assistance respiratoire.

En général, le gaz respiratoire est délivré par les lunettes durant les phases inspiratoires et les phases expiratoires ; durant les phases expiratoires, le gaz étant expiré par le patient en même temps que des gaz expirés riches en CO₂ provenant des poumons.

Actuellement, les interfaces respiratoires faciales connaissent une utilisation croissante liée, notamment, à leur efficacité et à la généralisation des techniques de ventilation artificielle non-invasives dans diverses applications hospitalières ou extra-hospitalières, tels les soins à domicile.

Bien que conçues à l'origine pour être de simples interfaces de traitement destinées à appliquer une pression ou un débit de gaz déterminé à un patient, celles-ci se sont très vite révélées être d'utiles moyens ou outils de diagnostic permettant de caractériser efficacement la respiration du patient.

A cette fin, on a très rapidement équipé ces interfaces de moyens complémentaires de diagnostic, en particulier de thermistances destinées à suivre le flux respiratoire nasal et/ou buccal du patient.

Classiquement, les thermistances utilisées jusqu'à présent se composent d'un capteur de température fixé à demeure sur l'interface.

Or, étant donné que chaque patient a une morphologie qui lui est propre, il a été constaté que la thermistance n'est pas toujours correctement positionnée par rapport à la bouche du patient.

Il en résulte alors un certain nombre de problèmes, notamment des contacts intempestifs entre la thermistance et la bouche du patient, ou, à l'inverse, une distance trop importante entre elles, conduisant, dans un cas comme dans l'autre à des pertes de signal de flux buccal et donc à des imprécisions parfois importantes de la mesure pouvant conduire à des diagnostics erronés.

En outre, étant donné que les interfaces actuellement utilisées comprennent des thermistances fixées à demeure, se pose aussi le problème de pouvoir assurer une désinfection correcte de la thermistance d'un patient à l'autre.

Le but de la présente invention est alors de proposer une interface respiratoire faciale à thermistance ne présentant pas les inconvénients précités et qui soit simple de réalisation et d'utilisation.

La présente invention concerne alors une interface respiratoire faciale comprenant un corps d'interface portant au moins une thermistance ayant une extrémité active susceptible de venir se placer en regard de la bouche d'un utilisateur lorsque l'interface est mise en position sur la région faciale dudit utilisateur, caractérisée en ce que la longueur L séparant l'extrémité active du corps d'interface est ajustable, notamment par l'opérateur.

Dans le cadre de l'invention, par "extrémité active", on entend la partie de la thermistance venant se positionner en regard ou vis-à-vis de la bouche de l'utilisateur de manière à pouvoir assurer la fonction de la thermistance en détectant d'éventuels flux respiratoires buccaux dudit utilisateur.

Selon le cas, l'interface respiratoire selon l'invention peut comprendre l'une ou plusieurs des caractéristiques suivantes :
- on éloigne ladite extrémité active du corps d'interface en opérant une traction sur ladite extrémité, de manière à augmenter la longueur L.
- le corps d'interface comporte un support portant ladite thermistance.
- le support comprend une partie allongée creuse au sein de laquelle la thermistance peut coulisser, de préférence une partie tubulaire.
- la partie support allongée et creuse comporte une fente longitudinale.
- la partie support allongée et creuse comporte une expansion formant un T.
- l'interface est choisie dans le groupe formé par les masques respiratoires et les lunettes respiratoires.
- elle est reliée, via une canalisation de gaz, à un appareil de ventilation artificielle.

Selon un autre aspect, l'invention concerne aussi un appareil respiratoire utilisable pour traiter ou diagnostiquer des troubles respiratoires du sommeil chez une personne, comprenant une interface respiratoire faciale selon l'invention ; selon le cas, l'appareil est du type à un ou à deux niveaux de pression.

L'invention va maintenant être décrite plus en détail à l'aide de figures annexées données à titre illustratif mais non limitatif.

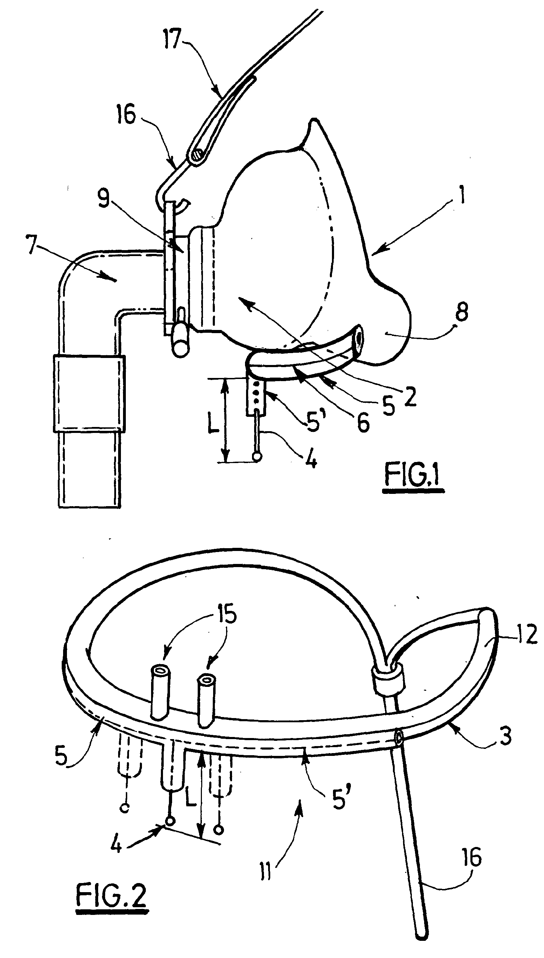

La figure 1 représente une vue générale de profil d'une interface 1 respiratoire faciale, ici un masque nasal respiratoire selon l'invention, lequel est destiné à être positionné sur la région nasale d'un utilisateur.

Plus précisément, le masque nasal comporte un corps 2 d'interface ou coquille définissant une chambre nasale, laquelle chambre nasale est conformée pour recouvrir au moins une partie de la région nasale de l'utilisateur, c'est-à-dire pour recouvrir au moins le nez de l'utilisateur. Le corps 3 comporte deux extensions latérales 8, symétriques et destinées à venir prendre appuis sur une partie du visage de l'utilisateur lorsque ledit masque est en position sur la région nasale de l'utilisateur, de manière à assurer et à améliorer et, d'une part, l'étanchéité, et d'autre part, le maintien en position du masque sur le visage.

De préférence, le corps 2 et les extensions 8 latérales sont réalisées en une pièce unique moulée, c'est-à-dire en une pièce monobloc en un matériau polymère, tel du silicone ou analogue.

En outre, le corps 2 de masque est dotée d'un orifice 9 d'alimentation en gaz respiratoire au niveau duquel orifice 9 d'alimentation sont agencés des moyens de connexion 7 permettant de raccorder le masque à une source de gaz sous pression, tel un ventilateur ou tout autre appareil d'assistance respiratoire délivrant un gaz respiratoire à un ou plusieurs niveau de pression.

Dans le cas présent, les moyens de connexion 7 comprennent un connecteur muni d'une bague de connexion, laquelle porte des moyens d'accrochage 16, de coopérer avec des harnais accrochés 17 et/ou des lanières et/ou des moyens analogues de fixation, pour permettre de modifier le site de fixation du harnais 17, en fonction de la morphologie de l'utilisateur, de manière à permettre un positionnement et un maintien efficace du masque sur le visage. Pour permettre un maintien efficace du masque sur le visage de l'utilisateur tout en répartissant l'effort de pression et donc améliorant le confort pour l'utilisateur, on choisit une dimension comprise entre 1 et 5 centimètres et une largeur comprise entre 1 et 4 centimètres pour chacune des extensions latérales 8.

Le masque nasal selon l'invention peut être directement relié, à un circuit de gaz acheminant un flux respiratoire issu d'une source de gaz respiratoire, tel un dispositif d'assistance respiratoire, jusqu'aux voies aériennes d'un patient.

En outre, on voit sur la figure 1, que le masque porte à sa partie inférieure un support 5 ayant une forme allongée tubulaire au sein duquel est agencé une thermistance 3 munie d'une extrémité active 4 destinée à être placée en regard de la bouche de l'utilisateur pour y assurer sa fonction, c'est-à-dire détecter les flux respiratoires buccaux.

Conformément à l'invention, la longueur L peut-être ajustée à volonté pour placer correctement la partie active 4 en regard de la bouche de l'utilisateur et ce, quelle que soit la morphologie de celui-ci.

L'ajustage en longueur se fait par traction, c'est-à-dire en tirant doucement d'un côté ou de l'autre de la thermistance, selon si l'on souhaite augmenter ou diminuer la longueur L.

Afin de permettre un démontage rapide de l'ensemble, notamment en vue d'un nettoyage efficace, la partie support 5 comporte une partie longitudinale 6 permettant d'extraire facilement la thermistance 3 pour procéder à son nettoyage et à sa désinfection ou, à l'inverse, sa réinsertion dans le support 5 tubulaire, après nettoyage.

En outre, comme on peut le voir, le support 5 comporte une extension 5 formant un "T" avec la partie allongée dudit support.

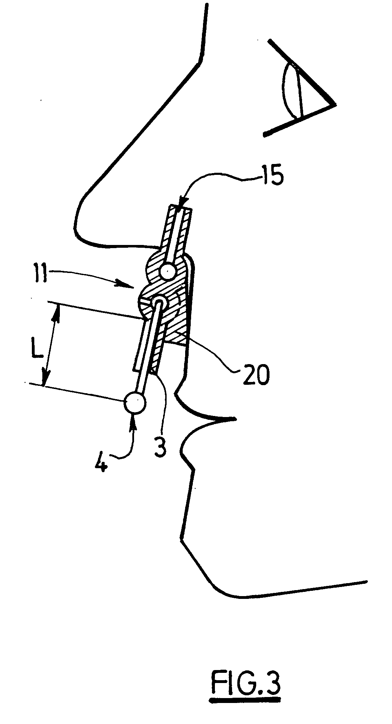

Les figures 2 et 3 représentent, quant à elles, un deuxième mode de réalisation d'une interface 11 respiratoire selon l'invention, dans lequel celle-ci à une forme de lunettes 11 respiratoires.

Ces lunettes respiratoires se composent d'un corps 12 de lunettes de forme tubulaire destiné à acheminer le gaz jusqu'aux narines du patient auquel le gaz est administré par l'intermédiaire de deux canules 15.

Le corps 12 porte à sa partie inférieure un support 5 tubulaire creux au sein duquel est insérée une thermistance 3 ayant, comme précédemment, une extrémité active 4 séparée du support d'une distance L ajustable ; la thermistance 3 étant, là encore, mobile en coulissement dans le support 5.

Afin de faciliter le montage et le démontage de l'ensemble, le support 5 est, comme précédemment, fendu 5' sur toute sa longueur.

La figure 3 représente les lunettes de la figure 2 en position sur le visage d'un utilisateur, ce qui permet de mieux comprendre l'intérêt de pouvoir faire varier, c'est-à-dire de modifier ou d'ajuster à volonté, la distance L pour pouvoir placer correctement l'extrémité 4 de la thermistance 3 en regard de 'la bouche de l'utilisateur.

Une interface respiratoire selon la présente invention peut être utilisée pour le traitement systématique de troubles respiratoires du sommeil, notamment de l'apnée du sommeil ou du ronflement.

## Revendications

1. Interface (1,11) respiratoire faciale comprenant un corps (2,12) d'interface portant au moins une thermistance (3) ayant une extrémité active (4) susceptible de venir se placer en regard de la bouche d'un utilisateur lorsque l'interface (1,11) est mise en position sur la région faciale dudit utilisateur, **caractérisée en ce que** la longueur (L) séparant l'extrémité active (4) du corps (2,12) d'interfaçe est ajustable.

2. Interface selon la revendication 1, **caractérisée en ce qu'**on éloigne ladite extrémité active (4) du corps d'interface (2,12) en opérant une traction sur ladite extrémité, de manière à augmenter la longueur (L).

3. Interface selon l'une des revendications 1 ou 2, **caractérisée en ce que** le corps d'interface (2,12) comporte un support (5) portant ladite thermistance (3).

4. Interface selon l'une des revendications 1 à 3, **caractérisée en ce que** le support (5) comprend une partie allongée creuse au sein de laquelle la thermistance peut coulisser, de préférence une partie tubulaire.

5. Interface selon l'une des revendications 3 ou 4, **caractérisée en ce que** la partie support allongée et creuse comporte une fente longitudinale (6).

6. Interface selon l'une des revendications 3 à 5, **caractérisée en ce que** la partie support allongée et creuse comporte une expansion (5) formant un T.

7. Interface selon l'une des revendications 1 à 6, **caractérisée en ce que** l'interface est choisie dans le groupe formé par les masques respiratoires et les lunettes respiratoires.

8. Interface selon l'une des revendications 1 à 7, **caractérisée en ce qu'**elle est reliée, via une canalisation de gaz, à un appareil de ventilation artificielle.

9. Appareil respiratoire utilisable pour traiter ou diagnostiquer des troubles respiratoires du sommeil chez une personne, comprenant une interface (1,11) respiratoire faciale selon l'une des revendications 1 à 8, de préférence ladite interface est reliée audit appareil par l'intermédiaire d'une ou plusieurs canalisations de gaz.

10. Appareil selon la revendication 9, **caractérisé en ce qu'**il est du type à un ou à deux niveaux de pression.
